# FASCICULE DE BREVET EUROPEEN

(11) **EP 0 842 938 B1**
(45) Date de publication et mention de la délivrance du brevet: **12.05.1999**
(21) Numéro de dépôt: 97402601.5
(22) Date de dépôt: 31.10.1997
(51) Int. Cl.: C07F 7/08, C07F 7/21, C07D 311/36, A61K 7/42

(54) **Nouveaux dérivés siliciés de flavones filtres, compositions cosmétiques photoprotectrices les contenant et utilisations**
Silylierte Flavonderivate als Filter, diese enthaltende kosmetische Sonnenschutzzusammensetzungen und deren Verwendung
Silated filtering flavone derivatives, cosmetic sunscreen compositions containing them and their use

(30) Priorité: 19.11.1996 FR 9614097
(43) Date de publication de la demande: 20.05.1998
(73) Titulaire: L'OREAL, 75008 Paris (FR)
(72) Inventeur: Leduc, Madeleine, Résidence Les Chèvrefeuilles, 75011 Paris (FR); Richard, Hervé, 93420 Villepinte (FR); Lagrange, Alain, 77700 Coupuray (FR)
(74) Mandataire: Miszputen, Laurent

(56) Documents cités:
- DE-A-19 508 608

## Description

L'invention concerne de nouveaux dérivés siliciés de flavones, liposolubles, photostables et présentant un excellent pouvoir d'absorption dans le domaine des rayonnements UV-A. L'invention concerne également des compositions, notamment cosmétiques, contenant ces nouveaux dérivés, qui peuvent être destinées à la photoprotection de la peau et/ou des cheveux contre le rayonnement UV, en particulier le rayonnement solaire, et/ou à la dépigmentation de la peau. L'invention concerne également l'utilisation de ces nouveaux composés dans des compositions anti-oxydantes.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel afin de contrôler ainsi la couleur de leur peau. Il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreux composés destinés à la photoprotection (UV-A et/ou UV-B) de la peau ont été proposés à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été préconisés à cet effet, on peut citer notamment les dérivés de flavone comme la quercetine et ses esters décrits dans JP 55-92305, JP 57-14517, JP 55-111411, JP 57-35506, DE 19508608. Cependant, ces substances ne présentent pas toutes les propriétés requises pour une utilisation convenable comme filtres UV dans les compositions antisolaires. La quercetine, en particulier, présente un caractère mutagène qui rend son utilisation peu souhaitable (cf. Special Issue of Mutation Research, Vol. 221, n°3, pages 165 à 180). La solubilité de ces molécules dans les différents types de formulations utilisés en matière de protection solaire n'est pas toujours très bonne (liposolubilité notamment) et leur incorporation dans les compositions cosmétiques est de ce fait fastidieuse. De plus, leur pouvoir filtrant intrinsèque peut encore paraître insuffisant, elles peuvent ne pas posséder une stabilité suffisante à la lumière (photostabilité) et elles peuvent également présenter une mauvaise résistance à l'eau et à la sueur. Il est également souhaitable que ces substances filtrantes ne pénètrent pas dans la peau.

La présente invention vise à résoudre les problèmes ci-dessus en proposant de nouveaux dérivés siliciés de flavones qui présentent des propriétés améliorées, notamment au niveau de leur solubilité dans les corps gras et de leur stabilité à la lumière.

Plus précisément encore, il a été trouvé, selon la présente invention, qu'en greffant sur une chaîne siliconée un ou plusieurs groupements flavone il était possible d'aboutir à de nouveaux composés obviant aux inconvénients des filtres de l'art antérieur, ces nouveaux composés présentant, outre des propriétés filtrantes très élevées, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet. De plus, certains de ces composés peuvent être utilisés à titre d'agent dépigmentant ou d'agent anti-oxydant.

La présente invention a ainsi pour premier objet de nouveaux composés qui sont caractérisés par le fait qu'ils sont constitués d'une chaîne siliconée comportant au moins une unité de formule (1) : 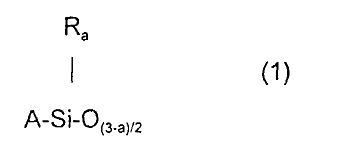 ou qu'ils répondent à la formule (2) suivante :

A-SiR'₁R'₂R'₃ (2)

dans lesquelles :
- R désigne un groupe hydrocarboné saturé ou insaturé en C₁-C₃₀, un groupe hydrocarboné halogéné en C₁-C₈ ou un groupe triméthylsilyloxy,
- a est égal à 1 ou 2,
- R'₁, R'₂, R'₃ , identiques ou différents, sont choisis parmi les radicaux alkyles et alcényles en C₁-C₈, linéaires ou ramifiés, saturés ou insaturés,
- A est un radical de formule (I) suivante : 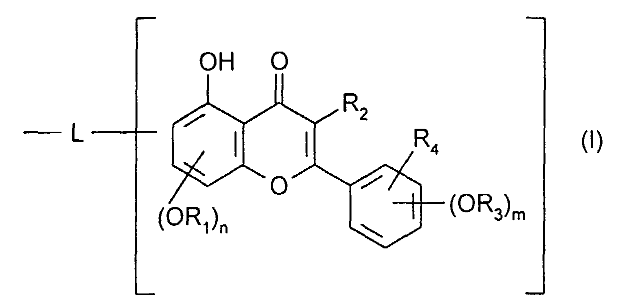 dans laquelle :
- L est un radical divalent permettant l'accrochage du radical A sur la chaîne siliconée,
- les radicaux R₁ et R₃, identiques ou différents, représentent indépendamment un atome d'hydrogène, un radical alkyle en C₁-C₁₀ linéaire ou ramifié ou un radical alcènyle en C₂-C₈ linéaire ou ramifié, deux OR₁ adjacents sur le noyau aromatique pouvant former ensemble un groupement alkylidène dioxy dans lequel le groupe alkylidène contient de 1 à 2 atomes de carbone,
- R₂ représente un atome d'hydrogène, un radical hydroxy, un radical alcoxy en C₁-C₁₀ linéaire ou ramifié ou un radical phényle éventuellement substitué,
- R₄ est un atome d'hydrogène ou un radical alkyle en C₁-C₈,
- n et m sont indépendamment 0, 1 ou 2.

De préférence, L répond à l'une des formules (a) ou (a') suivantes : 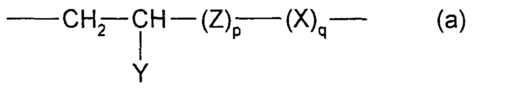 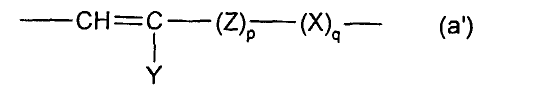 dans lesquelles :
- X représente O ou NH,
- Z est un radical alcane di-yle en C₁-C₆ linéaire ou ramifié, saturé ou insaturé, éventuellement substitué par un radical hydroxyle ou alcoyle en C₂-C₈, linéaire ou ramifié, saturé ou insaturé,
- Y représente un atome d'hydrogène, un radical hydroxyle ou un radical alkyle en C₁-C₈, linéaire ou ramifié, saturé ou insaturé,
- p et q sont 0 ou 1.

De préférence encore, les composés selon l'invention répondent à l'une des formules (3) ou (4) suivantes : 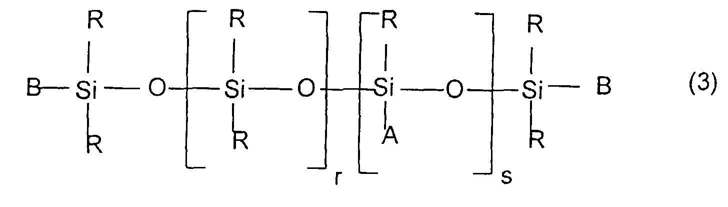 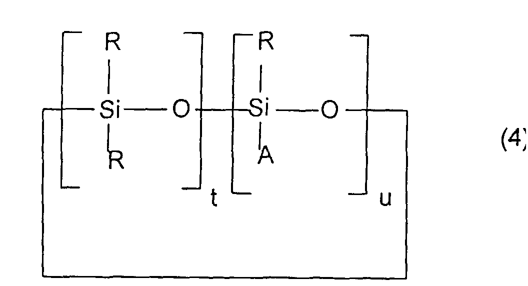 dans lesquelles :
- R désigne un groupe hydrocarboné saturé ou insaturé en C₁-C₃₀, un groupe hydrocarboné halogéné en C₁-C₈ ou un groupe triméthylsilyloxy,
- B, identiques ou différents, sont choisis parmi les radicaux R et le radical A,
- r est un nombre entier choisi entre 0 et 50 inclusivement,
- s est un nombre entier choisi entre 0 et 20 inclusivement et si s est 0, au moins un des deux symboles B est A,
- u est un nombre entier compris entre 1 et 6 inclus,
- t est un nombre entier entre 0 et 10 inclus,
- t + u est égal ou supérieur à 3.

Les composés de l'invention présentent une excellente liposolubilité et peuvent ainsi être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. De plus, leurs propriétés cosmétiques sont très bonnes, à savoir en particulier que ces produits, par rapport aux flavones de l'art antérieur, sont moins collantes et apportent plus de douceur.

En outre, les composés de l'invention présentent un excellent pouvoir filtrant intrinsèque à l'égard des rayonnements ultraviolet UV-A et UV-B.

Ces nouveaux dérivés siliciés de flavones peuvent ainsi être utilisés comme filtres solaires pour la peau humaine et les cheveux. Ils peuvent aussi être utilisés comme agents protecteurs de la lumière dans l'industrie des plastiques.

De préférence, les radicaux R, identiques ou différents sont choisis parmi les radicaux alkyles en C₁-C₁₀, linéaires ou ramifiés, saturés ou insaturés, le radical phényle et le radical trifluoro-3,3,3 propyle, au moins 80% en nombre des radicaux R étant le radical méthyle.

Dans les formules (1) à (4) ci-dessus, on préfère plus particulièrement les dérivés statistiques ou bien définis à blocs présentant au moins l'une des caractéristiques suivantes :
- R est méthyle,
- B est méthyle,
- r est compris entre 0 et 3 inclus,
- s est compris entre 1 et 3 inclus,
- t + u est compris entre 3 et 5,
- R'₁, R'₂, R'₃ sont méthyle.

Parmi les composés ci-dessus, on préfère plus particulièrement ceux pour lesquels r est compris entre 0 et 3 inclus et s est compris entre 1 et 3 inclus.

De préférence, le radical A présente au moins l'une, et encore plus préférentiellement l'ensemble, des caractéristiques suivantes :
- R₂ est H, OH ou méthoxy,
- p est 1,
- R₄ est H ou ter-butyl,
- Y est méthyle,
- n = m = 0,
- Z est le radical divalent -CH₂-.

Parmi les composés préférés de l'invention, on peut citer plus particulièrement le composé de formule (3) répondant à l'ensemble des caractéristiques suivantes :
- r = 0,
- s = 1,
- B est méthyle,
- R est méthyle,
- Y est méthyle,
- q = n = m = 0,
- R₂ est hydrogène,
- R₄ est le radical t.Butyl,
- p = 1,
- Z est le radical divalent -CH₂-,

ou encore le composé de formule (3) répondant à l'ensemble des caractéristiques suivantes :
- r = 0,
- s = 1,
- B est méthyle,
- R est méthyle,
- Y est méthyle,
- n = m = 0,
- q = 1,
- X est l'oxygène,
- R₂ est l'hydrogène,
- R₄ est l'hydrogène,
- p = 1,
- Z est le radical divalent -CH₂-.

De plus, les dérivés possédant des radicaux OH libres peuvent être utilisés comme agent dépigmentant ou anti-oxydant. Dans ce cas, on préfère les composés de l'invention pour lesquels au moins un radical R₁ ou R₃ représente l'hydrogène.

Pour préparer les dérivés de formules (1) à (4), on peut procéder classiquement en mettant en oeuvre une réaction d'hydrosilylation à partir du dérivé siloxanique ou silanique correspondant dans lequel, par exemple, tous les radicaux A sont des atomes d'hydrogène. Ce dérivé est dénommé par la suite dérivé à SiH.

Les groupes SiH peuvent être présents dans la chaîne et/ou aux extrémités de la chaîne. Ces dérivés à SiH sont des produits bien connus dans l'industrie des silicones et sont généralement disponibles dans le commerce. Ils sont par exemple décrits dans les brevets américains US-A-3 220 972, US-A-3 697 473 et US-A-4 340 709.

Les dérivés à SiH correspondant aux composés de formules (2), (3) et (4) peuvent être donc représentés par les formules (5) à (7) suivantes :

H-SiR'₁R'₂R'₃ (5)

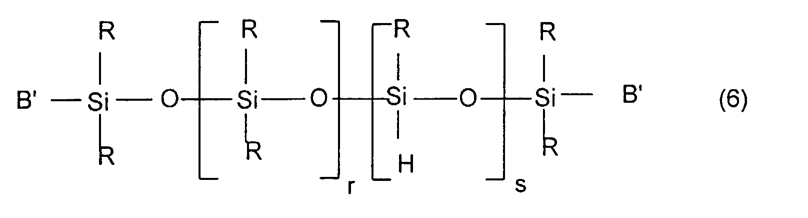

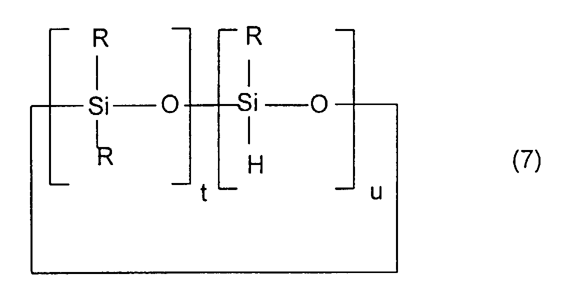

dans lesquelles :
- R'₁, R'₂ et R'₃ ont la signification donnée ci-dessus pour la formule (2),
- R, r, s, t et u ont la signification donnée ci-dessus pour les formules (3) et (4),
- B', identiques ou différents, sont choisis parmi les radicaux R et un atome d'hydrogène.

Afin de préparer les composés de l'invention de formules (2) à (4) ci-dessus, on procède de la manière suivante : sur le dérivé à SiH de formules (5), (6) ou (7), on effectue une réaction d'hydrosilylation en présence d'une quantité catalytiquement efficace d'un catalyseur au platine sur un dérivé organique de flavone choisi parmi ceux de formule (I') suivante : 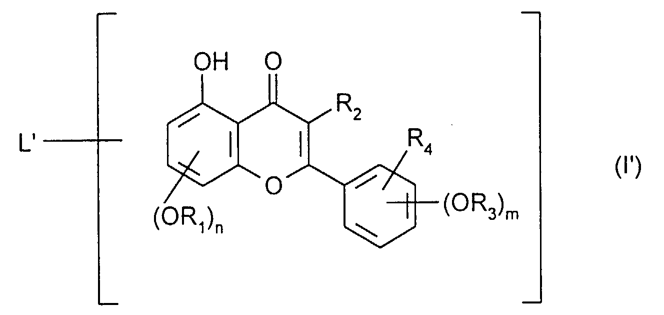 dans laquelle R₁, R₂, R₃, R₄, n et m ont la même signification qu'à la formule (I) ci-dessus et L' répond à l'une des deux formules (b) et (b') suivantes : 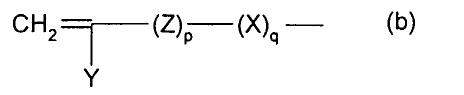 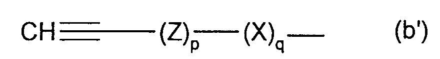 dans lesquelles X, Y, Z, p et q ont les mêmes significations qu'aux formules (a) et (a') ci-dessus.

La réaction d'hydrosilylation s'effectue donc selon l'une des deux réactions suivantes : 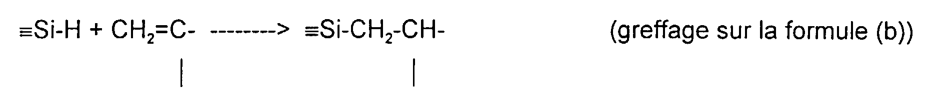 ou

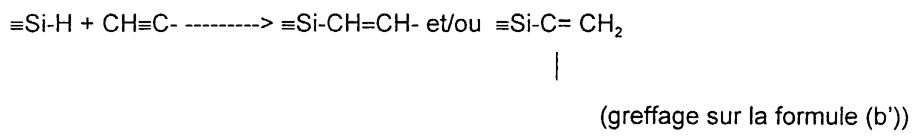

Dans le cas particulier où le composé de formule (2), (3) ou (4) que l'on cherche à préparer présente au moins un radical R₁ ou R₃ qui représente l'hydrogène, on procède de la manière suivante : avant de réaliser la réaction d'hydrosilylation décrite ci-dessus, on protège la fonction OH correspondant au groupement OR₁ ou OR₃ en la silylant au préalable par un groupement triméthylsilyl, par réaction par exemple avec le chloro triméthyl silane.

On réalise alors la réaction d'hydosilylation décrite ci-dessus puis on traite les composés obtenus par une base telle que la soude 0,5 N afin de déprotéger le groupement OR₁ ou OR₃ pour lequel R₁ ou R₃ était l'hydrogène en substituant un hydrogène au groupement triméthylsilyl. On obtient ainsi les composés de formules (2) à (4) pour lesquels au moins un radical R₁ ou R₃ est l'hydrogène.

Comme dérivés de flavone utilisables pour la préparation des composés selon l'invention, on préfère tout particulièrement ceux répondant à la formule (I') ci-dessus et présentant au moins l'une, et préférentiellement l'ensemble, des caractéristiques suivantes :
- R₂ est H, OH ou méthoxy,
- p est 1,
- R₄ est H ou ter.butyl,
- Y est méthyle,
- n = m = 0,
- Z est le radical divalent -CH₂-.

La présente invention a ainsi également pour objet les composés répondant à la formule (I") suivante : 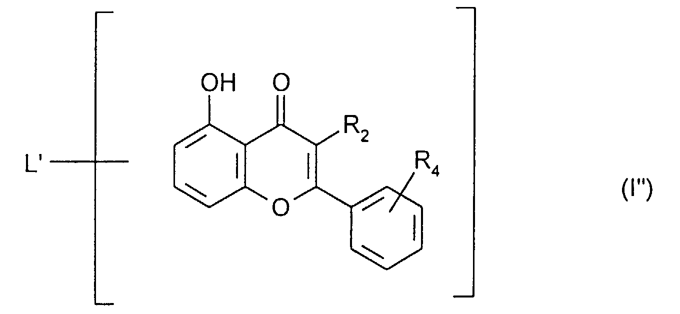 dans laquelle :
- R₂ est H, OH ou méthoxy,
- R₄ est H ou ter.butyl,
- et L' est le radical de formule suivante : 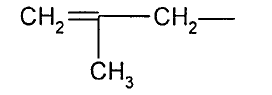 utiles comme intermédiaires de synthèse des composés de formules (1) à (4) selon l'invention.

Comme dérivés de flavone convenant particulièrement bien à la préparation des composés selon l'invention, on peut citer la 5-hydroxy-7-(2-méthylallyloxy) flavone, produit provenant de l'allylation de la chrysine commerciale ou encore la 2-(4-tert-butyl phenyl)-5-hydroxy chromen-4-one obtenue par condensation de la 2,6-dihydroxyacétophénone avec deux moles de chlorure de l'acide p-t.butyl benzoïque, puis transposition du produit résultant en 2,6-dihydroxy-4'-tert-butyldibenzoylméthane en présence de soude (réarrangement de Baker-Venkataraman), et enfin cyclisation en présence d'acide acétique.

Les composés de formule (I') peuvent également être facilement obtenus par alkylation par des halogénures d'alcènes ou d'alcynes de dérivés de quercetine connus et répondant à la formule (c) suivante : 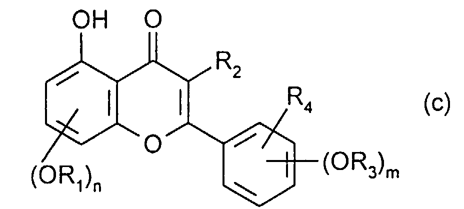 dans laquelle R₁, R₂, R₃, R₄, n et m ont la même signification qu'aux formules (a) et (b) ci-dessus.

On peut aussi utiliser des dérivés de flavone correspondant aux composés de formule (I') ci-dessus qui existent à l'état naturel tels que :
- la 8-(1,1-dimethylallyl) galangin,
- le 8-(1,1-dimethylallyl) kaempferride,
- le 8-(1,1-dimethylallyl) kaempférol,
qui sont des produits provenant des bourgeons de Platanus acerifolia (cf. Heterocycles, Vol. 43, N° 2, 1996).

Une autre voie de préparation des dérivés de formule (2) pour lesquels X est l'oxygène consiste à partir du dérivé de formule (c) suivante : 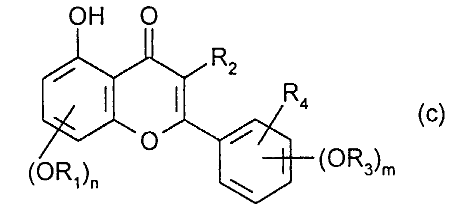 dans laquelle au moins un radical R₁ ou R₃ est l'hydrogène, les autres radicaux, R₁, R₂, R₃, R₄, n et m ayant la même signification qu'aux formules (a) et (b) ci-dessus et à lui faire réagir un dérivé silanique de formule (8) suivante :

Hal-(Z)ₚ-CHY-CH₂-SiR'₁R'₂R'₃ (8)

dans laquelle Hal représente un halogène et plus particulièrement le chlore et les radicaux Y, Z, R'₁, R'₂ R'₃ et p ont les mêmes significations que ci-dessus.

Ainsi, par exemple, si dans la formule (c) ci-dessus, un radical R₁ est l'hydrogène, on obtient alors un dérivé silanique de flavone correspondant à la formule (9) suivante : 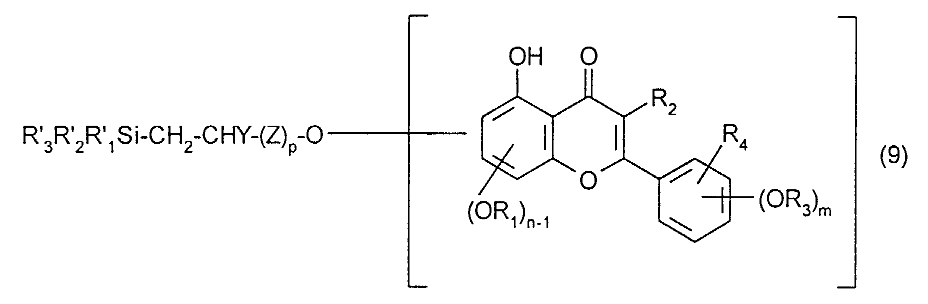 dans laquelle les significations de R₁, R₂, R₃, R₄, R'₁, R'₂, R'₃, Y et Z sont les mêmes que ci-dessus.

La présente invention a également pour objet une composition comprenant un composé de formule (1) à (4) selon l'invention dans un support approprié. Le support peut être par exemple une composition de matière plastique. Il peut également être approprié pour une application topique. Dans ce cas, la composition selon l'invention est une composition cosmétique qui comprend un support cosmétiquement acceptable.

De préférence, la composition selon l'invention est une composition destinée à protéger une matière sensible au rayonnement ultraviolet, en particulier au rayonnement solaire, comprenant une quantité efficace d'au moins un composé conforme à l'invention. Dans une forme préférée de réalisation de l'invention, cette composition est destinée à protéger la peau et/ou les cheveux.

Les composés de formule (1), (2), (3) ou (4) sont généralement présents dans la composition de l'invention dans des proportions comprises entre 0,1 % et 20 % en poids, de préférence entre 0,5 % et 10 % en poids, par rapport au poids total de la composition.

Les compositions selon l'invention peuvent bien entendu contenir un ou plusieurs filtres solaires complémentaires actifs dans l'UVA et/ou l'UVB (absorbeurs), autres que les composés conformes à la présente invention, hydrophiles ou lipophiles. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions selon l'invention peuvent encore contenir des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Elle peut contenir les adjuvants cosmétiques habituellement utilisés dans le domaine cosmétique, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des séquestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanoline hydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

La composition cosmétique de l'invention peut être utilisée comme composition protectrice de l'épiderme humain ou des cheveux contre les rayons ultraviolets, comme composition antisolaire ou comme produit de maquillage, comme composition dépigmentante ou anti-oxydante.

Cette composition peut se présenter en particulier sous forme de lotion, de lotion épaissie, de gel, de crème, de lait, de poudre, de bâtonnet solide et eventuellement être conditionnée en aérosol et se présenter sous forme de mousse ou de spray.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est plus particulièrement destinée à la dépigmentation de la peau, elle peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a encore pour objet l'utilisation d'un composé conforme à l'invention dans des, ou pour la fabrication de, compositions destinées à protéger des matières sensibles au rayonnement ultraviolet, en particulier au rayonnement solaire.

L'invention a encore pour objet l'utilisation d'un composé de formule (1), (2), (3) ou (4) conforme à l'invention pour la préparation d'un médicament destiné à prévenir les effets néfastes des rayonnements UV.

L'invention a encore pour objet l'utilisation d'un composé de formule (1), (2), (3) ou (4) conforme à l'invention à titre d'agent filtrant les rayonnements UV, en particulier pour contrôler la couleur de la peau.

La présente invention a également pour objet l'utilisation d'un composé de formule (1), (2), (3) ou (4) ci-dessus pour lequel au moins un des radicaux R₁ ou R₃ est l'hydrogène, à titre d'agent dépigmentant dans des compositions cosmétiques.

La présente invention a encore pour objet l'utilisation d'un composé de formule (1), (2), (3) ou (4) ci-dessus pour lequel au moins un des radicaux R₁ ou R₃ est l'hydrogène, à titre d'agent anti-oxydant dans des compositions cosmétiques.

L'invention a enfin pour objet un procédé non thérapeutique de protection de la peau et/ou des cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1), (2), (3) ou (4) tel que défini ci-avant.

L'invention a finalement pour objet un procédé non thérapeutique de contrôle de la variation de couleur de la peau due aux rayonnements UV, qui consiste à appliquer sur la peau une quantité efficace de la composition cosmétique définie ci-dessus, ou d'un composé de formule (1), (2), (3) ou (4) tel que défini ci-avant.

La présente invention a également pour objet un procédé cosmétique de dépigmentation et/ou blanchiment de la peau humaine, consistant à appliquer sur la peau pigmentée une composition comprenant une quantité efficace d'un composé de formule (1), (2), (3) ou (4) ci-dessus dans laquelle au moins un des radicaux R₁ ou R₃ est l'hydrogène.

Les exemples qui suivent illustrent l'invention sans toutefois en limiter la portée.

### EXEMPLE 1 :

### Préparation de la 2-(4-tert-butylphényl)-5-hydroxy-6-[2-méthyl-3-[1,3,3,3-tétraméthyl-1-[(triméthylsilyl)oxy]disiloxanyl]propyl]-chromen-4-one :

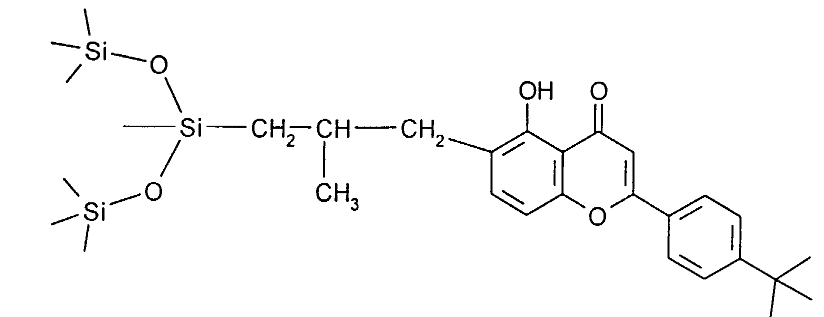

### a) Première étape : préparation de la 2,6-di-(4 -tert-butyl-benzoyloxy)acétophénone:

On chauffe à 60-70°C une solution de 2,6-dihydroxy acétophénone (11,4 g, 0,075 mole) dans 50 ml de pyridine. On y ajoute goutte à goutte en 20 minutes de l'acide para-tert-butyl benzoïque (31 g, 0,158 mole). Le mélange est chauffé à 70-80°C pendant 2 heures puis il est versé dans un mélange de 300 ml d'eau et de 50 ml d'acide chlorhydrique concentré. Le solide formé est filtré, lavé à l'eau et recristallisé dans 200 ml d'éthanol. On obtient 29 g d'une poudre beige de 2,6-di-(4-tert-butyl-benzoyloxy)-acétophénone (Rendement : 82 %).

### b) Deuxième étape : préparation du 1-(4-tert-butyl-phenyl)-3-(2,6-dihydroxyphenyl)-propane-1,3-dione :

Dans une solution du dérivé précédent (25 g, 0,053 mole) dans 208 ml de pyridine, on ajoute à température ambiante et par portions en 15 minutes 28 g de soude en pastilles. La température monte jusqu'à 32°C. On laisse sous agitation et à température ambiante pendant 12 heures puis on le verse dans un mélange de 500 ml d'eau glacée et de 250 ml d'acide chlorhydrique concentré. Après extraction avec du chloroforme, séchage de la phase organique et concentration, on récupère 20 g de 1-(4-tert-butyl-phenyl)-3-(2,6-dihydroxy-phenyl)-propane-1,3-dione.

### c) Troisième étape : préparation du 2-(4-tert-butyl phenyl)-5-hydroxy chromen-4-one :

Le dérivé précédent (10 g, 0,032 mole) est dissout dans 85 ml d'acide acétique et le mélange est traité au reflux pendant 2 heures. Cette solution est versée dans 500 ml d'eau et le précipité formé est filtré. On récupère une poudre jaune pâle de 2-(4-tert-butyl phenyl)-5-hydroxy chromen-4-one.

### d) Quatrième étape : préparation du 2-(4-tert-butyl phenyl)-5-(2-méthyl-allyloxy)-chromen-4-one :

Dans un mélange du dérivé précédent (1,45 g, 0,005 mole) et de carbonate de potassium (0,73g, 0,0055 mole) dans 15 ml de DMF porté à 80°C, on ajoute goutte à goutte en 20 minutes du chlorure de méthallyle (1 g, 0,011 mole). On laisse 10 heures à 80°C. On refroidit et verse le mélange réactionnel dans de l'eau. Le précipité formé est filtré, lavé à l'eau et recristallisé dans l'éthanol. On obtient 1,35 g d'une poudre blanche de 2-(4-tert-butyl phenyl)-5-(2-méthylallyloxy)-chromen-4-one dont les caractéristiques sont les suivantes :
- Pf : 142-143 °C

| Analyse élémentaire pour C₂₃ H₂₄ O₃ | | | |
|---|---|---|---|
| théorie | C : 79.28 | H : 6.94 | O : 13.78 |
| trouvé | C : 79.21 | H : 7.11 | O : 13.70 |

### e) Cinquième étape : préparation du 2-(4-tert-butyl phenyl)-5-hydroxy-6-(2-méthyl-allyl)-chromen-4-one :

Le dérivé précédent (0,9 g) est chauffé pendant 3 heures à 180 °C. Après refroidissement, le mélange réactionnel est chromatographié sur silice (éluant : dichlorométhane). On obtient ainsi 0,75 g d'une poudre jaune de 2-(4-tert-butyl phenyl)-5-hydroxy-6-(2-méthyl-allyl)-chromen-4-one dont les caractéristiques sont les suivantes :
- Pf : 137-138 °C
- UV (Ethanol) λₘₐₓ = 280 nm, εₘₐₓ = 31 200
λₘₐₓ = 308 nm, εₘₐₓ = 19 200
λₘₐₓ = 345 nm, εₘₐₓ = 7 280

| Analyse élémentaire pour C₂₃ H₂₄ O₃ | | | |
|---|---|---|---|
| théorie | C : 79.28 | H : 6.94 | O : 13.78 |
| trouvé | C : 79.16 | H : 7.01 | O : 13.76 |

### f) Sixième étape : préparation du dérivé de l'exemple 1 :

A une solution du dérivé précédent (0,7 g, 0,002 mole) et de catalyseur (complexe à 3-3.5% poids de Pt dans du cyclovinylméthylsiloxane de Hüls Petrarch PC085 : 10 µl) dans 1 ml de toluène sec porté à 80°C, on ajoute goutte à goutte en 20 minutes 0,49 g (0,0022 mole) d'heptaméthyltrisiloxane. On laisse à cette température pendant 3 heures. On concentre le mélange réactionnel et on obtient après chromatographie sur silice (éluant : dichlorométhane) 1 g d'une poudre jaune pâle du dérivé de l'exemple 1 dont les caractéristiques sont les suivantes :
- Pf : 79-80 °C
- UV (Ethanol) λₘₐₓ = 281 nm, εₘₐₓ = 32 600
λₘₐₓ = 307 nm, εₘₐₓ = 19 400
λₘₐₓ = 344 nm, εₘₐₓ = 6 800

| Analyse élémentaire pour C₃₀ H₄₆ O₅ Si₃ | | | |
|---|---|---|---|
| théorie | C : 63.11 | H : 8.12 | Si : 14.76 |
| trouvé | C : 63.25 | H : 8.12 | Si : 14.90. |

### EXEMPLE 2 :

### Préparation de la 2-phényl-5-hydroxy-7-[2-méthyl-3-[1,3,3,3-tétraméthyl-1-(triméthylsilyl)oxy]disiloxanyl]propyloxy]-chromen-4-one :

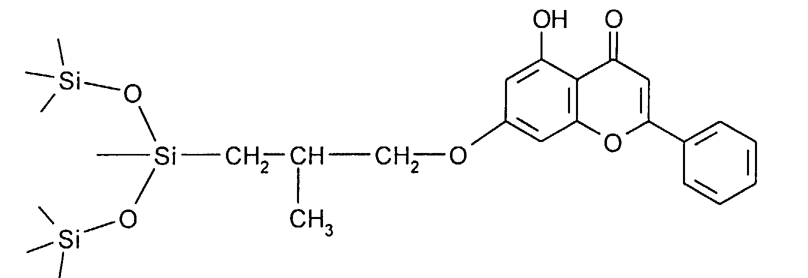

### a) Première étape : préparation du 2-phényl-5-hydroxy-7-(2-methyl-allyl) chromen-4-one :

Dans un mélange de 2-phényl-5,7-dihydroxy chromen-4-one (12,7 g, 0,05 mole) et de carbonate de potassium (15,2g, 0,11 mole) dans 50 ml de DMF porté à 80°C, on ajoute goutte à goutte en 20 minutes du chlorure de méthallyle (9,96 g, 0,11 mole). On laisse 4 heures à 80°C. On refroidit et verse le mélange réactionnel dans de l'eau glacée. Le précipité formé est filtré et lavé à l'eau. On obtient après chromatographie sur silice (éluant : dichlorométhane) 3,8 g d'une poudre jaune pâle de 2-phényl-5-hydroxy-7-(2-methyl-allyl) chromen-4-one dont les caractéristiques sont les suivantes :
- Pf : 138-140 °C

| Analyse élémentaire pour C₁₉ H₁₆ O₄ | | | |
|---|---|---|---|
| théorie | C : 74.01 | H 5.23 | O : 20.76 |
| trouvé | C : 73.97 | H : 5.39 | O : 20.51 |

### b) Deuxième étape : préparation du dérivé de l'exemple 2 :

A une solution du dérivé précédent (3,08 g, 0,01 mole) et de catalyseur (complexe à 3-3.5% poids de Pt dans du cyclovinylméthylsiloxane de Hüls Petrarch PC085 : 20 µl) dans 5 ml de toluène sec porté à 80°C, on ajoute goutte à goutte en 20 minutes 2,45 g (0,011 mole) d'heptaméthyltrisiloxane. On laisse à cette température pendant 4 heures. On concentre le mélange réactionnel et on obtient après recristallisation dans le méthanol 3 g d'une poudre blanc cassé du dérivé de l'exemple 2 dont les caractéristiques sont les suivantes :
- Pf : 54 °C
- UV (Ethanol) λₘₐₓ = 310 nm, εₘₐₓ = 12 750

| Analyse élémentaire pour C₂₆ H₃₈ O₆ Si₃ | | | |
|---|---|---|---|
| théorie | C : 58.83 | H : 7.22 | Si : 15.87 |
| trouvé | C : 58.77 | H :7.15 | Si : 16.10 |

### EXEMPLE 3 :

On donne ci-après un exemple concret d'une composition cosmétique antisolaire conforme à la présente invention, à savoir une crème antisolaire :
- composé de l'exemple 2 4 %
- mélange d'alcool cétylstéarylique et d'alcool cétylstéarylique oxyéthyléné (33 OE) vendu sous la dénomination commerciale « SINNOVAX AO » par HENKEL 7 %
- mélange de mono et distéarate de glycérol non autoémulsifiable 2 %
- alcool cétylique 1,5 %
- benzoate d'alcools en C₁₂-C₁₅ vendu sous la dénomination commerciale « FINSOLV TN » par WITCO 20 %
- polydiméthylsiloxane 1,5 %
- glycérine 17,5 %
- parfum, conservateur qs
- eau qsp 100 %

Cette crème est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40°C on ajoute enfin parfum et conservateur.

On obtient ainsi une crème antisolaire particulièrement efficace contre les UV B.

## Revendications

1. Composé constitué d'une chaîne siliconée comportant au moins une unité de formule (1): 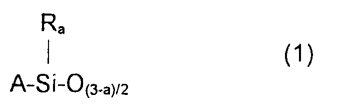 ou répondant à la formule (2) suivante :
A-SiR'₁R'₂R'₃ (2)
dans lesquelles :
- R désigne un groupe hydrocarboné saturé ou insaturé en C₁-C₃₀, un groupe hydrocarboné halogéné en C₁-C₈ ou un groupe triméthylsilyloxy,
- a est égal à 1 ou 2,
- R'₁, R'₂, R'₃, identiques ou différents, sont choisis parmi les radicaux alkyles et alcényles en C₁-C₈, linéaires ou ramifiés, saturés ou insaturés,
- A est un radical de formule (I) suivante : 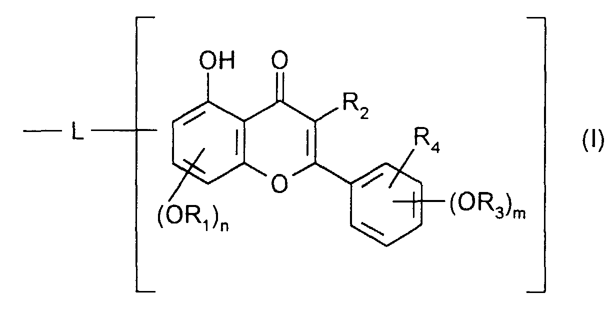 dans laquelle :
- L est un radical divalent permettant l'accrochage du radical A sur la chaîne siliconée,
- les radicaux R₁ et R₃, identiques ou différents, représentent indépendamment un atome d'hydrogène, un radical alkyle en C₁-C₁₀ linéaire ou ramifié ou un radical alcènyle en C₂-C₈ linéaire ou ramifié, deux OR₁ adjacents sur le noyau aromatique pouvant former ensemble un groupement alkylidène dioxy dans lequel le groupe alkylidène contient de 1 à 2 atomes de carbone,
- R₂ représente un atome d'hydrogène, un radical hydroxy, un radical alcoxy en C₁-C₁₀ linéaire ou ramifié ou un radical phényle éventuellement substitué,
- R₄ est un atome d'hydrogène ou un radical alkyle en C₁-C₈,
- n et m sont indépendamment 0, 1 ou 2.

2. Composé selon la revendication 1, caractérisé par le fait que le radical L répond à l'une des formules (a) ou (a') suivantes : 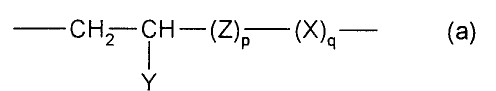 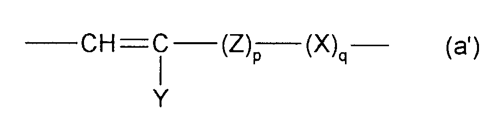 dans lesquelles :
- X représente O ou NH,
- Z est un radical alcane di-yle en C₁-C₆ linéaire ou ramifié, saturé ou insaturé, éventuellement substitué par un radical hydroxyle ou alcoyle en C₂-C₈, linéaire ou ramifié, saturé ou insaturé,
- Y représente un atome d'hydrogène, un radical hydroxyle ou un radical alkyle en C₁-C₈, linéaire ou ramifié, saturé ou insaturé,
- p et q sont 0 ou 1.

3. Composé selon l'une des revendications 1 ou 2, caractérisé par le fait qu'il répond à l'une des formules (3) ou (4) suivantes : 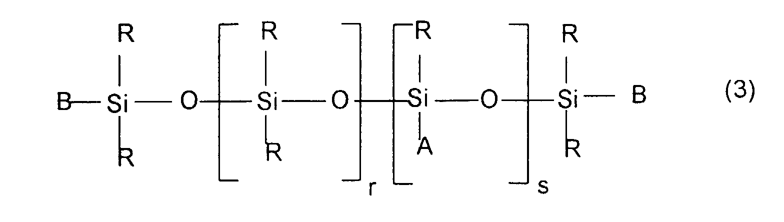 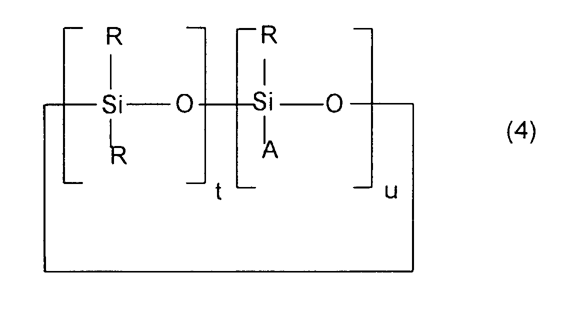 dans lesquelles :
- R désigne un groupe hydrocarboné saturé ou insaturé en C₁-C₃₀, un groupe hydrocarboné halogéné en C₁-C₈ ou un groupe triméthylsilyloxy,
- B, identiques ou différents, sont choisis parmi les radicaux R et le radical A,
- r est un nombre entier choisi entre 0 et 50 inclusivement,
- s est un nombre entier choisi entre 0 et 20 inclusivement et si s est 0, au moins un des deux symboles B est A,
- u est un nombre entier compris entre 1 et 6 inclus,
- t est un nombre entier entre 0 et 10 inclus,
- t + u est égal ou supérieur à 3.

4. Composé selon la revendication 3, caractérisé par le fait qu'il répond à la formule (3) et qu'il présente les deux caractéristiques suivantes :
- r est compris entre 0 et 3 inclus,
- s est compris entre 1 et 3 inclus.

5. Composé selon la revendication 4, caractérisé par le fait qu'il est choisi parmi la 2-(4-ter.butylphényl)-5-hydroxy-6-[2-méthyl-3-[1,3,3,3-tétraméthyl-1-[(triméthylsilyl)oxy]disiloxanyl]propyl]-chromen-4-one et la 2-phényl-5-hydroxy-7-[2-méthyl-3-[1,3,3,3-tétraméthyl-1(triméthylsilyl)oxy]disiloxanyl]propyloxy]-chromen-4-one.

6. Procédé de préparation des composés définis à l'une quelconque des revendications 1 à 5, caractérisé par le fait qu'il comprend l'étape suivante :
- on effectue, en présence d'une quantité catalytiquement efficace d'un catalyseur au platine, l'hydrosilylation d'un composé de formule (b) ou (b') suivantes : 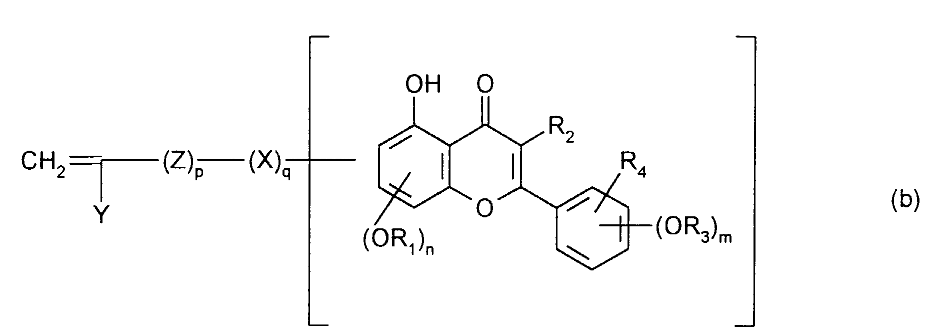 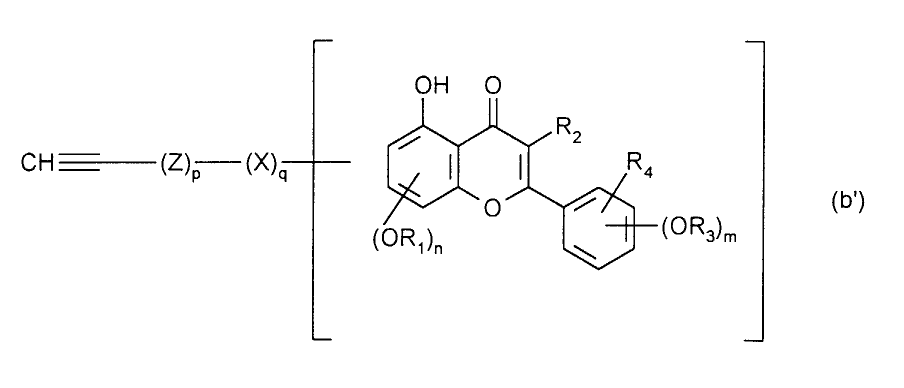 dans lesquelles R₁, R₂, R₃, R₄, X, Y, Z, n, m, p et q ont la même signification qu'à qu'à la revendication 1,
- par un dérivé SiH.

7. Procédé selon la revendication 6, caractérisé par le fait que le dérivé SiH est un composé répondant à l'une des formules (5) à (7) suivantes :
H-SiR'₁R'₂R'₃ (5) 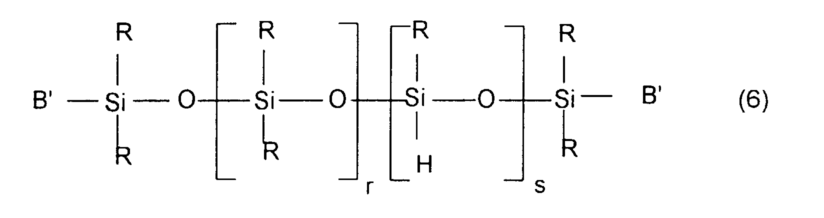 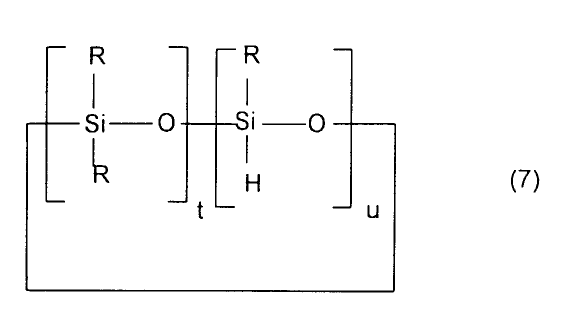
dans lesquelles :
- R'₁, R'₂ et R'₃ ont la signification donnée à la revendication 1 pour la formule (2),
- R, r, s, t et u ont la signification donnée à la revendication 2 pour les formules (3) et (4),
- B', identiques ou différents, sont choisis parmi les radicaux R et un atome d'hydrogène.

8. Procédé selon la revendication 6 ou 7, caractérisé par le fait que le composé de formule (b) ou (b') est obtenu par alkylation par des halogénures d'alcènes ou d'alcynes de dérivés de quercetine connus et répondant à la formule (c) suivante : 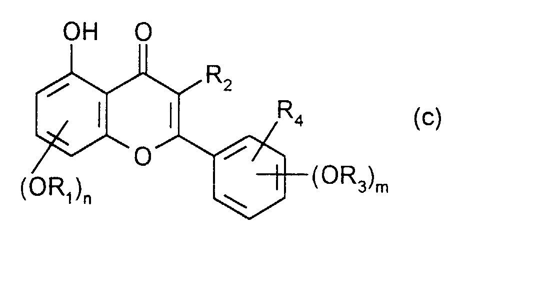 dans laquelle R₁, R₂, R₃, R₄, n et m ont la même signification que pour la formule (I) de la revendication 1.

9. Procédé de préparation des composés définis par la formule (2) à la revendication 1 pour lesquels X est l'oxygène, caractérisé par le fait qu'il comprend l'étape suivante :
- sur un composé de formule (c) suivante : 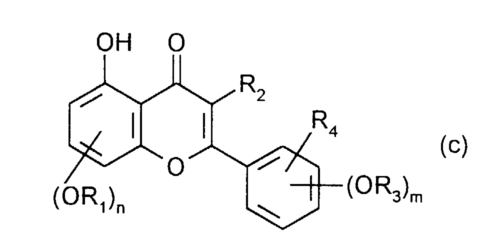 dans laquelle au moins un radical R₁ ou R₃ est l'hydrogène, les autres radicaux, R₁, R₂, R₃, R₄, n et m ayant la même signification qu'à la revendication 1, on fait réagir un dérivé silanique de formule (8) suivante ;
Hal-(Z)ₚ-CHY-CH₂-SiR'₁R'₂R'₃ (8)
dans laquelle Hal représente un halogène et plus particulièrement le chlore et les radicaux Y, Z, R'₁, R'₂ R'₃ et p ont les mêmes significations qu'à la revendication 1.

10. Composition cosmétique destinée à protéger la peau et/ou les cheveux du rayonnement UV comprenant dans un support cosmétiquement acceptable, au moins un composé tel que défini dans l'une quelconque des revendications 1 à 5.

11. Composition cosmétique destinée à la dépigmentation de la peau comprenant dans un support cosmétiquement acceptable, au moins un composé tel que défini dans l'une quelconque des revendications 1 à 5 pour lequel dans la formule (I) au moins un des radicaux R₁ ou R₃ est l'hydrogène.

12. Composition de matière plastique comprenant au moins un composé tel que défini dans l'une quelconque des revendications 1 à 5.

13. Composition selon l'une quelconque des revendications 10 à 12, caractérisée par le fait que le composé tel que défini à l'une quelconque des revendications 1 à 5 est présent dans la composition à une teneur allant de 0,1 à 20 % en poids, par rapport au poids total de la composition, de préférence allant de 0,5 % à 10 % en poids, par rapport au poids total de la composition.

14. Utilisation d'au moins un composé tel que défini dans l'une quelconque des revendications 1 à 5 pour la fabrication de compositions destinées à protéger des matières sensibles au rayonnement ultraviolet, en particulier au rayonnement solaire.

15. Utilisation d'au moins un composé tel que défini dans l'une quelconque des revendications 1 à 5 pour la préparation d'un médicament destiné à prévenir les effets néfastes des rayonnements UV.

16. Utilisation d'un composé tel que défini dans l'une quelconque des revendications 1 à 5 pour la fabrication d'une composition destinée à contrôler la variation de la couleur de la peau due aux rayonnement UV.

17. Utilisation d'un composé tel que défini dans l'une quelconque des revendications 1 à 5 pour lequel dans la formule (I) au moins un des radicaux R₁ ou R₃ est l'hydrogène pour la fabrication d'une composition destinée à la dépigmentation et/ou au blanchiment de la peau humaine.

18. Composés répondant à la formule (I") suivante : 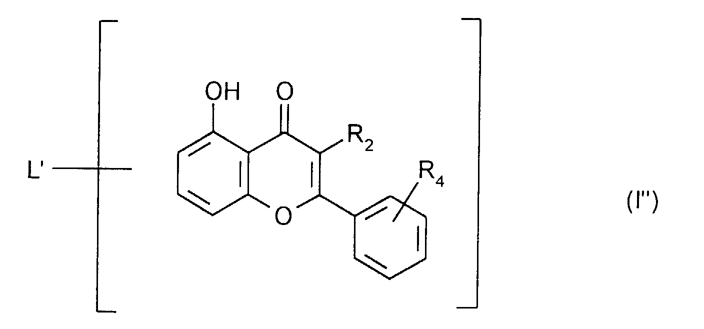 dans laquelle :
- R₂ est H, OH ou méthoxy,
- R₄ est H ou ter.butyl,
- et L' est le radical de formule suivante :

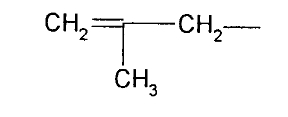

## Patentansprüche

1. Verbindung, die aus einer Siliconkette besteht, die mindestens eine Einheit der Formel (1) aufweist: 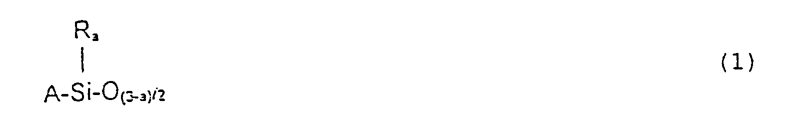 oder der folgenden Formel (2) entspricht:
A-SiR₁'R₂'R₃' (2)
worin:
- die Gruppe R eine gesättigte oder ungesättigte C₁₋₃₀-Kohlenwasserstoffgruppe, eine halogenierte C₁₋₈-Kohlenwasserstoffgruppe oder eine Trimethylsilyloxygruppe bedeutet,
- a 1 oder 2 bedeutet,
- die Gruppen R₁', R₂' und R₃', die identisch oder voneinander verschieden sind, unter den geradkettigen oder verzweigten, gesättigten oder ungesättigten Alkyl- und Alkenylgruppen mit 1 bis 8 Kohlenstoffatomen ausgewählt sind,
- die Gruppe A eine Gruppe der folgenden Formel (I) ist: 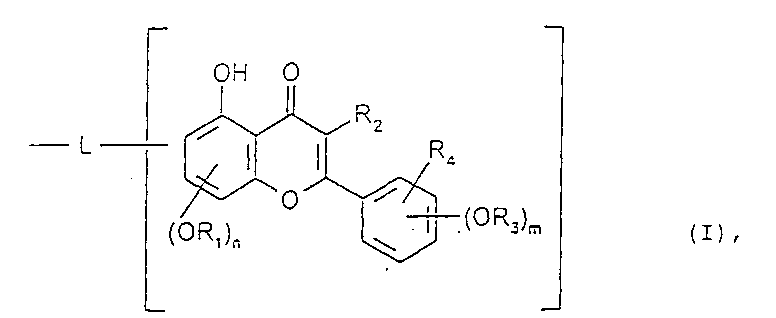 worin bedeuten:
- L eine zweiwertige Gruppe, die die Verknüpfung der Gruppe A mit der Siliconkette ermöglicht,
- die Gruppen R₁ und R₃, die identisch oder voneinander verschieden sind, unabhängig voneinander Wasserstoff, eine geradkettige oder verzweigte C₁₋₁₀-Alkylgruppe, eine geradkettige oder verzweigte C₂₋₈-Alkenylgruppe, wobei zwei am aromatischen Ring benachbarte Gruppen OR₁ gemeinsam eine Alkylidendioxygruppe bilden können, worin die Alkylidengruppe 1 bis 2 Kohlenstoffatome aufweist,
- R₂ Wasserstoff, Hydroxy, eine geradkettige oder verzweigte C₁₋₁₀-Alkoxygruppe oder eine gegebenenfalls substituierte Phenylgruppe,
- R₄ Wasserstoff oder eine C₁₋₈-Alkylgruppe,
- n und m unabhängig voneinander 0, 1 oder 2.

2. Verbindung nach Anspruch 1, dadurch gekennzeichnet, daß die Gruppe L einer der folgenden Formeln (a) oder (a') entspricht: 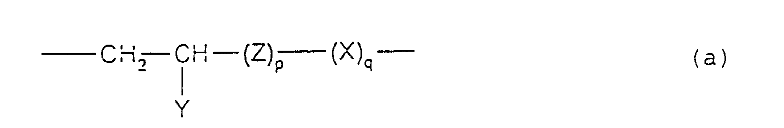 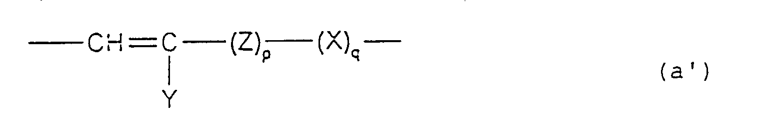 worin bedeuten:
- X O oder NH,
- Z eine geradkettige oder verzweigte, gesättigte oder ungesättigte Alkan-di-yl-Gruppe mit 1 bis 6 Kohlenstoffatomen, die gegebenenfalls mit einer Hydroxygruppe oder einer geradkettigen oder verzweigten, gesättigten oder ungesättigten C₂₋₈-Alkylgruppe substituiert ist,
- Y Wasserstoff, Hydroxy oder eine geradkettige oder verzweigte, gesättigte oder ungesättigte C₁₋₈-Alkylgruppe,
- p und q 0 oder 1.

3. Verbindung nach einem der Ansprüche 1 oder 2, dadurch gekennzeichnet, daß sie einer der folgenden Formeln (3) oder (4) entspricht: 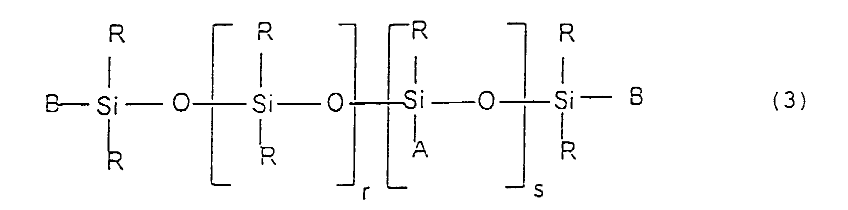 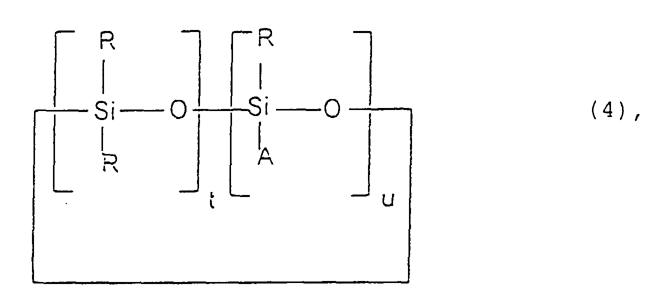 worin:
- die Gruppe R eine gesättigte oder ungesättigte C₁₋₃₀-Kohlenwasserstoffgruppe, eine halogenierte C₁₋₈-Kohlenwasserstoffgruppe oder eine Trimethylsilyloxygruppe bedeutet,
- die Gruppen B, die identisch oder voneinander verschieden sind, unter den Gruppen R oder der Gruppe A ausgewählt sind,
- r 0 oder eine ganze Zahl im Bereich von 1 bis einschließlich 50 bedeutet,
- s 0 oder eine ganze Zahl im Bereich von 1 bis einschließlich 20 bedeutet, wobei mindestens eine der beiden Gruppen B die Gruppe A bedeutet, wenn s 0 ist,
- u eine ganze Zahl im Bereich von 1 bis einschließlich 6 bedeutet,
- t 0 oder eine ganze Zahl im Bereich von 1 bis einschließlich 10 bedeutet, und
- t + u größer oder gleich 3 ist.

4. Verbindung nach Anspruch 3, dadurch gekennzeichnet, daß sie der Formel (3) entspricht und die beiden folgende Eigenschaften aufweist:
- r liegt im Bereich von 0 biseinschließlich 3,
- s liegt im Bereich von 1 bis einschließlich 3.

5. Verbindung nach Anspruch 5, dadurch gekennzeichnet, daß sie unter 2-(4-tert.-Butylphenyl)-5-hydroxy-6-[2-methyl-3-[1,3,3,3-tetramethyl-1-[(trimethylsilyl)oxy]disiloxanyl]propyl]-chromen-4-on und 2-Phenyl-5-hydroxy-7-[2-methyl-3-[1,3,3,3-tetramethyl-1-[(trimethylsilyl)oxy]-disiloxanyl]propyloxy]-chromen-4-on ausgewählt ist.

6. Verfahren zur Herstellung der Verbindungen nach einem der Ansprüche 1 bis 5, das folgenden Schritt umfaßt:
- Durchführung einer Hydrosilylierung mit einer Verbindung der folgenden Formel (b) oder (b') und einem SiH-Derivat in Gegenwart einer katalytisch wirksamen Menge eines Platinkatalysators: 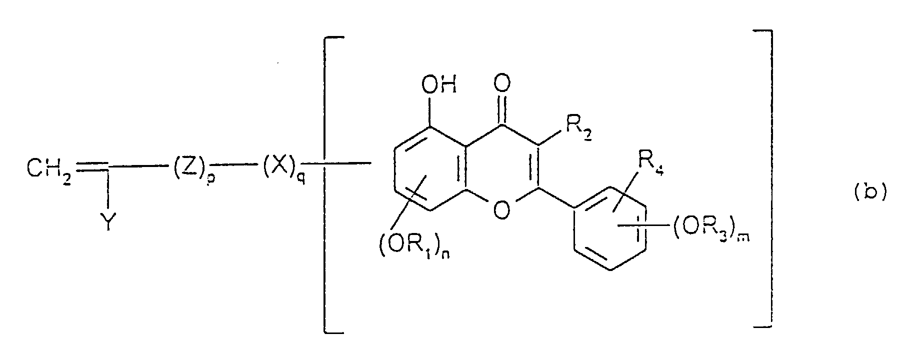 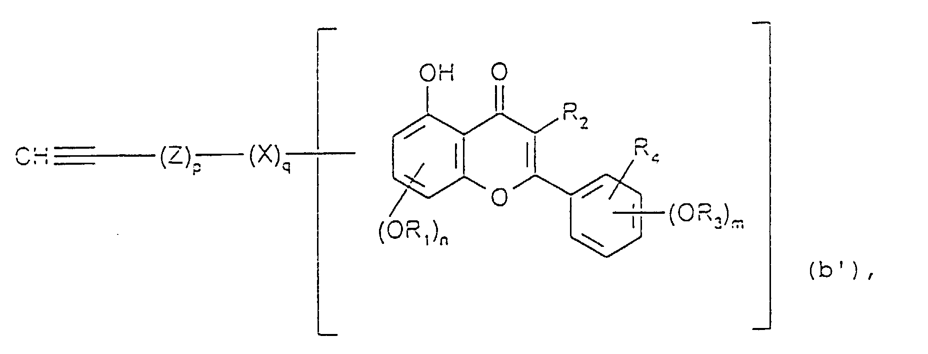 worin R₁, R₂, R₃, R₄, X, Y, Z, n, m, p und q die in Anspruch 1 angegebenen Bedeutungen aufweisen.

7. Verfahren nach Anspruch 6, dadurch gekennzeichnet, daß das SiH-Derivat eine Verbindung ist, die einer der folgenden Formeln (5) bis (7) entspricht:
H-SiR'₁R'₂R'₃ (5) 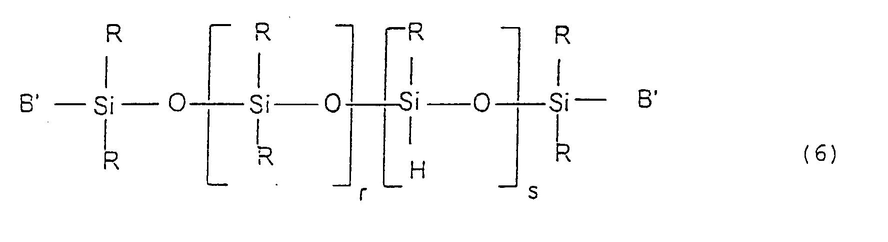 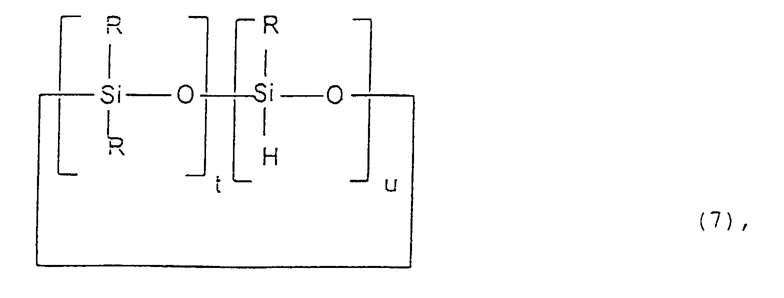
worin:
- R₁', R₂' und R₃' die in Anspruch 1 für Formel (2) angegebenen Bedeutungen aufweisen,
- R, r, s, t und u die in Anspruch 2 für Formel (3) und (4) angegebenen Bedeutungen ausweisen, und
- die Gruppen B', die identisch oder voneinander verschieden sind, unter den Gruppen R und Wasserstoff ausgewählt sind.

8. Verfahren nach Anspruch 6 oder 7, dadurch gekennzeichnet, daß die Verbindung der Formel (b) oder (b') durch Alkylierung der bekannten Quercetinderivate der folgenden Formel (c) mit Alken- oder Alkinhalogeniden hergestellt wird: 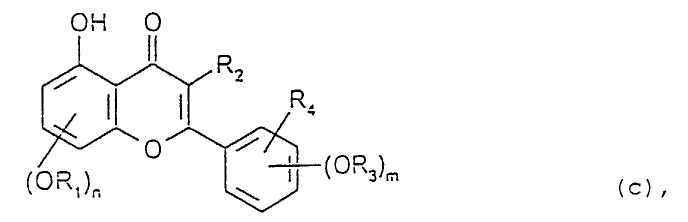 worin R₁, R₂, R₃, R₄, n und m die für die Formel (I) in Anspruch 1 angegebenen Bedeutungen aufweisen.

9. Verfahren zu Herstellung der Verbindungen der Formel (2) nach Anspruch 1, worin X Sauerstoff bedeutet, dadurch gekennzeichnet, daß es folgenden Schritt umfaßt:
- eine Verbindung der folgenden Formel (c): 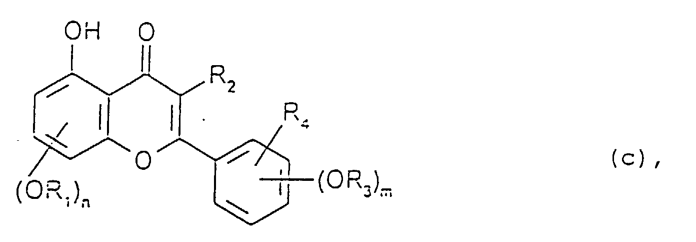 worin mindestens eine der Gruppen R₁ oder R₃ Wasserstoff bedeutet, wobei die anderen Gruppen R₁, R₂, R₃, R₄, n und m die in Anspruch 1 angegebenen Bedeutungen aufweisen,
wird mit einem Silanderivat der folgenden Formel (8) umgesetzt:
Hal-(Z)ₚ-CHY-CH₂-SiR'₁R'₂R'₃ (8),
worin Hal Halogen und insbesondere Chlor bedeutet und die Gruppen Y, Z, R'₁, R'₂, R'₃ und p die in Anspruch 1 angegebenen Bedeutungen aufweisen.

10. Kosmetische Zusammensetzung zum Schutz der Haut und/oder der Haare gegen UV-Strahlung, die in einem kosmetisch akzeptablen Träger mindestens eine Verbindung nach einem der Ansprüche 1 bis 5 enthält.

11. Kosmetische Zusammensetzung zur Depigmentierung der Haut, die in einem kosmetisch akzeptablen Träger mindestens eine Verbindung nach einem der Ansprüche 1 bis 5 enthält, worin in Formel (I) mindestens eine der Gruppen R₁ oder R₃ Wasserstoff bedeutet.

12. Zusammensetzung aus Kunststoff, die mindestens eine Verbindung nach einem der Ansprüche 1 bis 5 enthält.

13. Zusammensetzung nach einem der Ansprüche 10 bis 12, dadurch gekennzeichnet, daß die Verbindung nach eine der Ansprüche 1 bis 5 in der Zusammensetzung in einem Mengenanteil von 0,1 bis 20 Gew.-%, bezogen auf das Gesamtgewicht der Zusammensetzung, und vorzugsweise in einem Mengenanteil von 0,5 bis 10 Gew.-%, bezogen auf das Gesamtgewicht der Zusammensetzung, vorliegt.

14. Verwendung mindestens einer Verbindung nach einem der Ansprüche 1 bis 5 zur Herstellung von Zusammensetzungen, die dazu bestimmt sind, Materialien zu schützen, die gegenüber UV-Strahlung und insbesondere Sonnenlicht empfindlich sind.

15. Verwendung mindestens einer Verbindung nach einem der Ansprüche 1 bis 5 zur Herstellung eines Arzneimittel, das dazu bestimmt ist, den schädlichen Wirkungen der UV-Strahlung vorzubeugen.

16. Verwendung einer Verbindung nach einem der Ansprüche 1 bis 5 zur Herstellung eines Zusammensetzung, das dazu bestimmt ist, die durch UV-Strahlung hervorgerufene Farbänderung der Haut zu kontrollieren.

17. Verwendung einer Verbindung nach einem der Ansprüche 1 bis 5, wobei in Formel (I) mindestens eine der Gruppen R₁ oder R₃ Wasserstoff bedeutet, zur Herstellung eines Zusammensetzung, das dazu bestimmt ist, die menschliche Haut zu depigmentieren und/oder zu bleichen.

18. Verbindungen der folgenden Formel (I"): 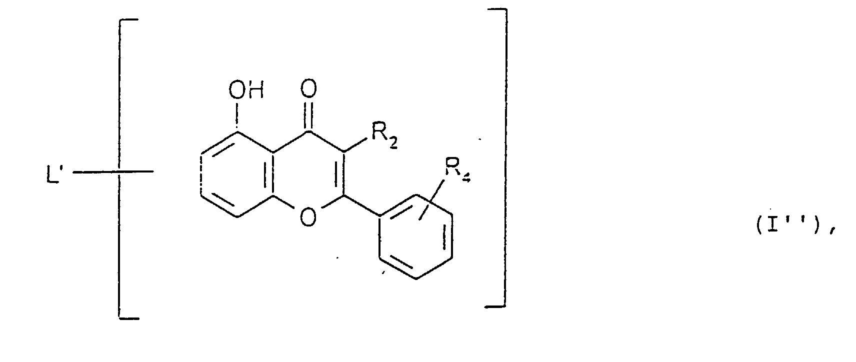 worin bedeuten:
- R₂ H, OH oder Methoxy,
- R₄ H oder tert.-Butyl,
- L' die Gruppe der folgenden Formel:

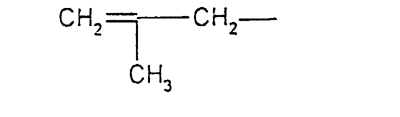

## Claims

1. Compound consisting of a silicone chain containing at least one unit of formula (1): 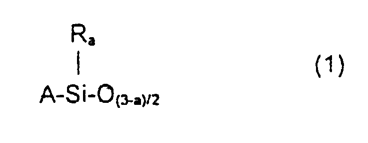 or corresponding to formula (2) below:
A-SiR'₁R'₂R'₃ (2)
in which:
- R denotes a saturated or unsaturated C₁-C₃₀ hydrocarbon-based group, a halogenated C₁-C₈ hydrocarbon-based group or a trimethylsilyloxy group,
- a is equal to 1 or 2,
- R'₁, R'₂ and R'₃, which may be identical or different, are chosen from linear or branched, saturated or unsaturated, C₁-C₈ alkyl and alkenyl radicals,
- A is a radical of formula (I) below: 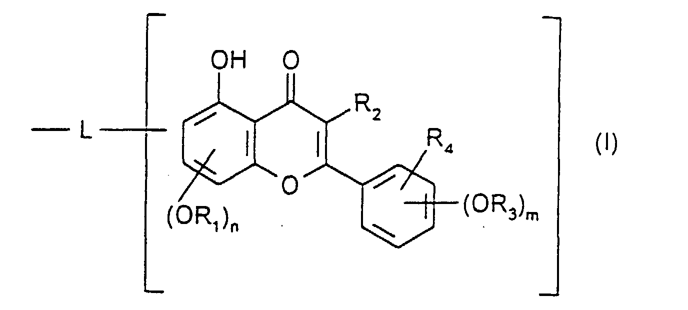 in which:
- L is a divalent radical which allows the radical A to be attached to the silicone chain,
- the radicals R₁ and R₃, which may be identical or different, independently represent a hydrogen atom, a linear or branched C₁-C₁₀ alkyl radical or a linear or branched C₂-C₈ alkenyl radical, it being possible for two adjacent OR1 groups on the aromatic ring to form together an alkylidenedioxy group in which the alkylidene group contains from 1 to 2 carbon atoms,
- R₂ represents a hydrogen atom, a hydroxyl radical, a linear or branched C₁-C₁₀ alkoxy radical or an optionally substituted phenyl radical,
- R₄ is a hydrogen atom or a C₁-C₈ alkyl radical,
- n and m are, independently, 0, 1 or 2.

2. Compound according to Claim 1, characterized in that the radical L corresponds to one of the formulae (a) and (a') below: 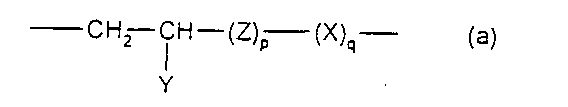 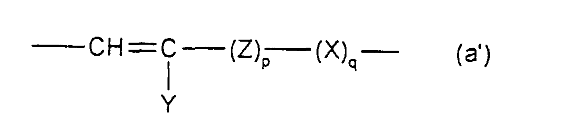 in which:
- X represents O or NH,
- Z is a linear or branched, saturated or unsaturated C₁-C₆ alkanediyl radical optionally substituted with a hydroxyl or a linear or branched, saturated or unsaturated, C₂-C₈ alkyl radical,
- Y represents a hydrogen atom, a hydroxyl radical or a linear or branched, saturated or unsaturated C₁-C₈ alkyl radical,
- p and q are 0 or 1.

3. Compound according to either of Claims 1 and 2, characterized in that it corresponds to one of the formulae (3) and (4) below: 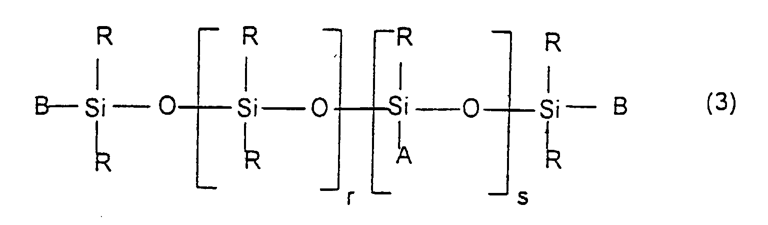 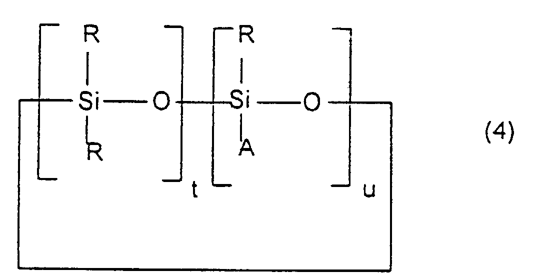 in which:
- R denotes a saturated or unsaturated C₁-C₃₀ hydrocarbon-based group, a halogenated C₁-C₈ hydrocarbon-based group or a trimethylsilyloxy group,
- B, which may be identical or different, are chosen from the radicals R and the radical A,
- r is an integer chosen between 0 and 50 inclusive,
- s is an integer chosen between 0 and 20 inclusive and, if s is 0, at least one of the two symbols B is A,
- u is an integer between 1 and 6 inclusive,
- t is an integer between 0 and 10 inclusive,
- t + u is greater than or equal to 3.

4. Compound according to Claim 3, characterized in that it corresponds to formula (3) and in that it has the following two characteristics:
- r is between 0 and 3 inclusive,
- s is between 1 and 3 inclusive.

5. Compound according to Claim 4, characterized in that it is chosen from 2-(4-tert-butylphenyl)-5-hydroxy-6-[2-methyl-3-[1,3,3,3-tetramethyl-1-[(trimethylsilyl)oxy]disiloxanyl]propyl]chromen-4-one and 2-phenyl-5-hydroxy-7-[2-methyl-3-[1,3,3,3-tetramethyl-1-(trimethylsilyl)oxy]disiloxanyl]propyloxy]chromen-4-one.

6. Process for preparing the compounds defined in any one of Claims 1 to 5, characterized in that it comprises the following step:
- hydrosilylation is carried out, in the presence of a catalytically effective amount of a platinum catalyst, on a compound of formula (b) or (b') below: 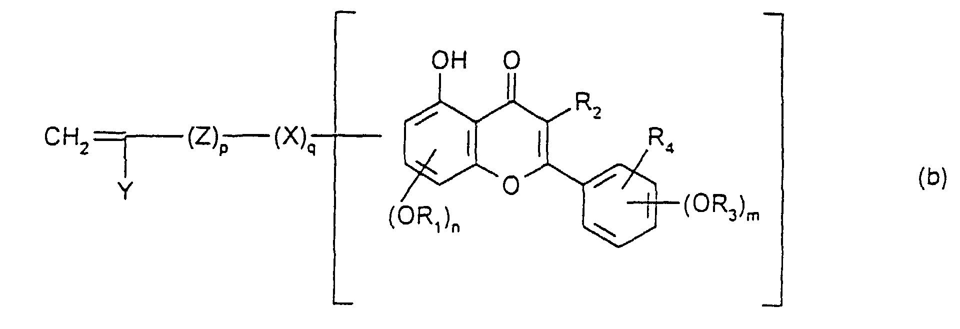 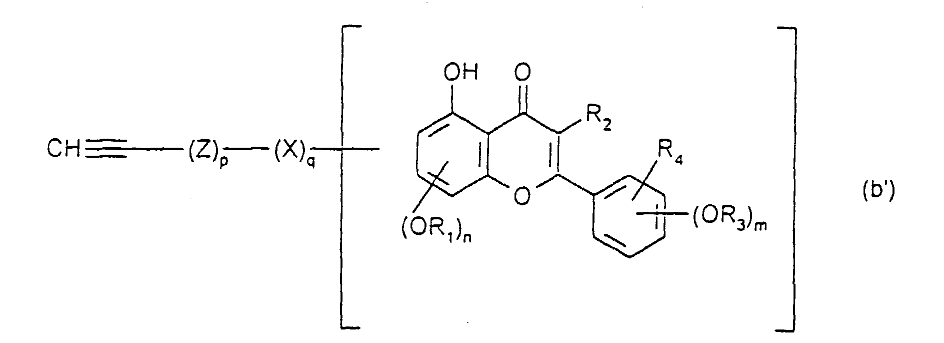 in which R₁, R₂, R₃, R₄, X, Y, Z, n, m, p and q have the same meaning as in Claim 1,
- with an SiH derivative.

7. Process according to Claim 6, characterized in that the SiH derivative is a compound corresponding to one of the formulae (5) to (7) below:
H-SiR'₁R'₂R'₃ (5) 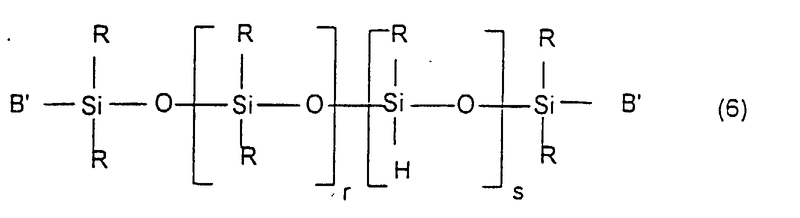 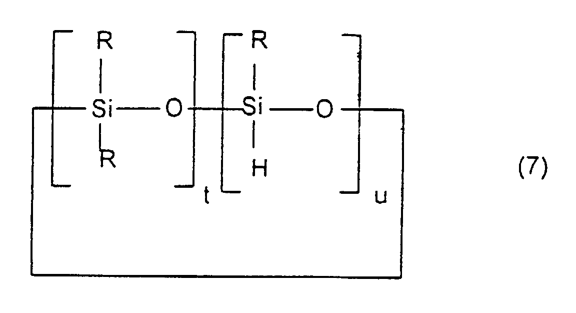
in which
- R'₁, R'₂ and R'₃ have the meaning given in Claim 1 for formula (2),
- R, r, s, t and u have the meaning given in Claim 2 for formulae (3) and (4),
- B', which may be identical or different, are chosen from the radicals R and a hydrogen atom.

8. Process according to Claim 6 or 7, characterized in that the compound of formula (b) or (b') is obtained by alkylation with halides of alkenes or of alkynes of known quercetin derivatives and corresponding to formula (c) below: 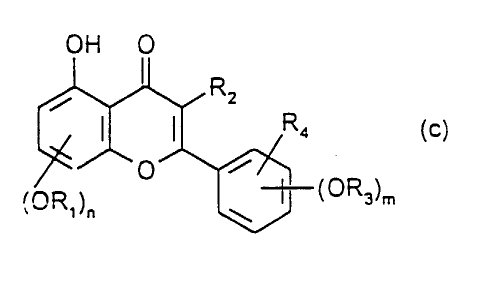 in which R₁, R₂, R₃, R₄, n and m have the same meaning as in formula (I) of Claim 1.

9. Process for preparing the compounds described by formula (2) in Claim 1, for which X is oxygen, characterized in that it comprises the following step:
- a compound of formula (c) below: 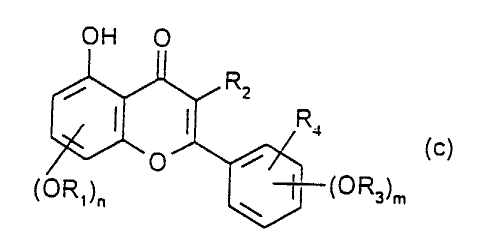 in which at least one radical R₁ or R₃ is hydrogen, the other radicals R₁, R₂, R₃, R₄, n and m having the same meaning as in Claim 1, is reacted with a silane derivative of formula (8) below:
Hal-(Z)ₚ-CHY-CH₂-SiR'₁R'₂R'₃ (8)
in which Hal represents a halogen and more particularly chlorine and the radicals Y, Z, R'₁, R'₂, R'₃ and p have the same meanings as in Claim 1.

10. Cosmetic composition intended for protecting the skin and/or the hair against UV radiation, comprising, in a cosmetically acceptable support, at least one compound as defined in any one of Claims 1 to 5.

11. Cosmetic composition intended for depigmenting the skin, comprising, in a cosmetically acceptable support, at least one compound as defined in any one of Claims 1 to 5 for which, in formula (I), at least one of the radicals R₁ and R₃ is hydrogen.

12. Plastic composition comprising at least one compound as defined in any one of Claims 1 to 5.

13. Composition according to any one of Claims 10 to 12, characterized in that the compound as defined in any one of Claims 1 to 5 is present in the composition at a content ranging from 0.1 to 20% by weight, relative to the total weight of the composition, preferably ranging from 0.5% to 10% by weight, relative to the total weight of the composition.

14. Use of at least one compound as defined in any one of Claims 1 to 5 for the manufacture of compositions intended for protecting substances that are sensitive to ultraviolet radiation, in particular to solar radiation.

15. Use of at least one compound as defined in any one of Claims 1 to 5 for the preparation of a medicament intended for preventing the harmful effects of UV radiation.

16. Use of a compound as defined in any one of Claims 1 to 5 for the manufacture of a composition intended for controlling the variation in skin colour due to UV radiation.

17. Use of a compound as defined in any one of Claims 1 to 5 for which, in formula (I), at least one of the radicals R₁ and R₃ is hydrogen, for the manufacture of a composition intended for depigmenting and/or bleaching human skin.

18. Compounds corresponding to formula (I") below: 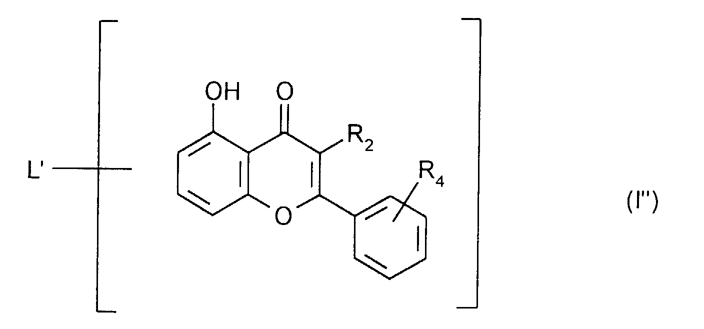 in which:
- R₂ is H, OH or methoxy,
- R₄ is H or tert-butyl, and
- L' is the radical of the following formula:
